# EUROPEAN PATENT APPLICATION

(11) **EP 4 699 555 A1**
(43) Date of publication of application: **25.02.2026**
(21) Application number: 25196904.4
(22) Date of filing: 20.08.2025
(51) Int. Cl.: A61B 18/14, A61B 5/287, A61B 18/00

(54) **COATED CATHETER ELECTRODES EMBEDDED IN A SLEEVE**

(30) Priority: 21.08.2024 US 202418810594
(71) Applicant: Biosense Webster (Israel) Ltd., 2066717 Yokneam (IL)
(72) Inventor: GOVARI, Assaf, 2066717 Yokneam (IL)
(74) Representative: Carpmaels & Ransford LLP

(57) **Abstract**

A medical probe includes a shaft, a basket assembly, and heat shrink sleeves. The shaft is configured for insertion into an organ of a patient. The basket assembly is mounted at a distal end of the shaft, the basket assembly comprising a plurality of splines. The plurality of electrodes is fitted on the plurality of splines, each electrode defining an outer conductive surface. The heat shrink sleeves are fitted over the electrodes, the sleeves comprising open windows that expose conductive surfaces of the electrodes.

## Description

### FIELD OF THE DISCLOSURE

The present disclosure relates generally to invasive medical probes, and particularly to cardiac catheters.

### BACKGROUND OF THE DISCLOSURE

Basket catheters typically have an elongated catheter body and a basket-shaped electrode assembly mounted at the distal end of the catheter body. The assembly has proximal and distal ends and comprises a plurality of splines connected at their proximal and distal ends. Each spline comprises at least one electrode that can be used for sensing and/or electrical ablation.

The present disclosure will be more fully understood from the following detailed description of the examples thereof, taken together with the drawings, in which:

### BRIEF DESCRIPTION OF THE DRAWINGS

Fig. 1 is a schematic pictorial illustration of an expandable basket assembly comprising electrode embedding sleeves, in accordance with an example of the present disclosure;
Fig. 2 is a schematic pictorial illustration of the expandable basket assembly comprising electrode embedding sleeves, in accordance with another example of the present disclosure;
Fig. 3 is a schematic pictorial illustration of the expandable basket assembly comprising electrode embedding sleeves that expose both the front and back sides of the electrodes, in accordance with an example of the present disclosure; and
Fig. 4 is a flow chart that schematically illustrates a method of forming sleeve-embedded electrodes of the basket catheter of Figs. 1-3, in accordance with an example of the present disclosure.

### DETAILED DESCRIPTION OF EXAMPLES

### OVERVIEW

Multi-electrode catheters (e.g., basket-shaped, loop-shaped, and others) that can perform therapeutic electrical ablation of a wall tissue of an organ typically include bulky electrodes (e.g., with a 1 mm diameter) capable of delivering sufficient ablative energy. One ablative technique used by such catheters is pulsed field ablation (PFA) that establishes an electric field for ablation.

The electrodes are mounted on the basket's splines (or on a spiral arm, in the case of a loop catheter), and, typically, each electrode protrudes or bulges around the spline or arm on which it is mounted.

When deploying the catheter's distal end out of a sheath, or retracting the distal end back into the sheath, the bulging electrodes may suffer shear forces. Such forces may also occur when sliding the electrodes along the wall tissue. The shear forces can loosen the electrodes bonded to the spline. In addition, the shear forces may also lead to the peeling of a coating, e.g., an impedance-enhancing polymer coating, on the outer surface of the electrodes. Moreover, it may be difficult to slide the assembly into and out of the sheath, which a smoother catheter structure with the sleeves may ease.

Examples of the present disclosure that are described herein add a heat shrink sleeve over the electrodes and open a window to expose the electrodes' outer conductive surface. The sleeves provide a smooth transition from the diameter of the spline to the diameter of the electrodes. The smooth transition reduces the shear forces when moving into and out of the sheath, thereby preventing shear forces from loosening the electrodes. The thickness of the sleeve provides a raised edge around the window to protect the coating from being scratched.

In some examples, epoxy glue is injected into the sleeve to bond the electrodes to the spline, which also helps form a smooth inclination between the spline and the upper surface of the electrode.

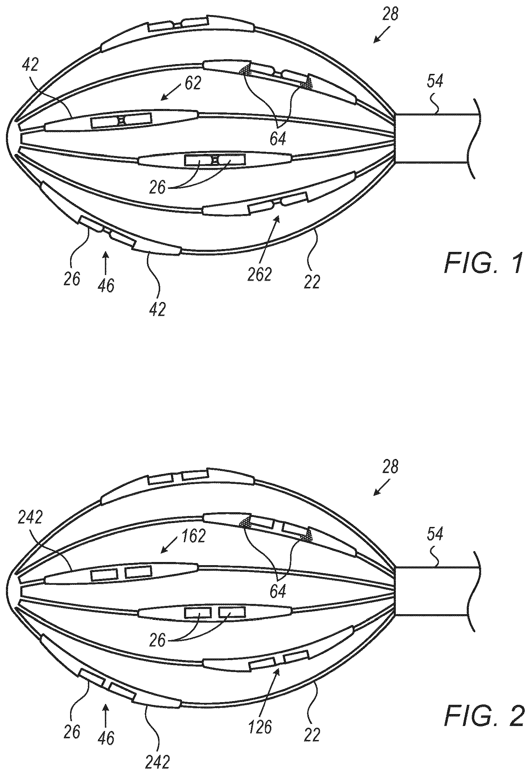

In some applications, the sleeve is also configured to selectively insulate the back surface of the electrode, i.e., the surface facing the inner volume of the basket. This assists in directing an electric field generated between electrodes to the volume surrounding the basket. The technique provides a sleeve configured for these applications, as seen in Fig. 1 below.

In different examples, seen in Figs 1 and 2, either a pair of electrodes or single electrodes are exposed through windows in the sleeves.

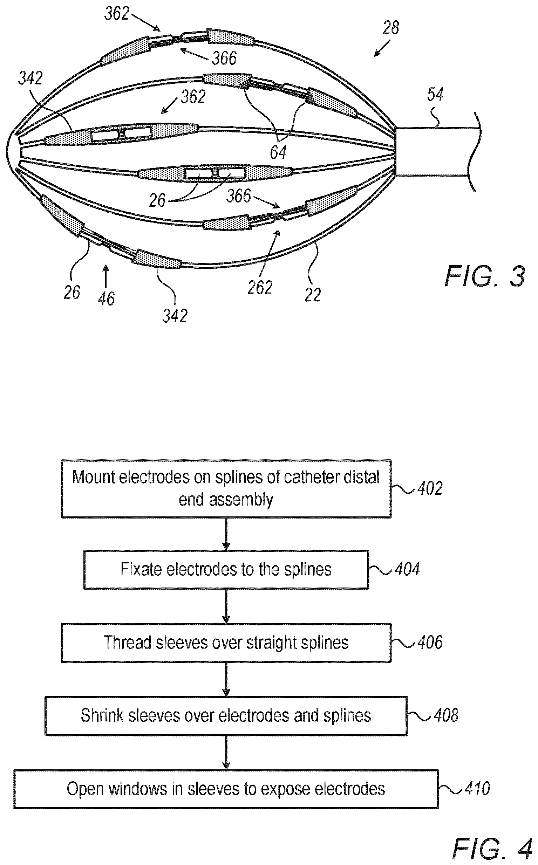

In other applications, it might be useful to expose the electrode's back surface. The technique also provides a sleeve configured for these applications, as seen in Fig. 3 below.

The sleeve arrangement may also improve the bonding integrity between the electrode and the spline, thus enhancing the basket's durability against shear forces during catheter deployment and retraction.

### COATED CATHETER ELECTRODES EMBEDDED IN A SLEEVE

Fig. 1 is a schematic pictorial illustration of a basket expandable assembly 28 comprising electrode-embedding sleeves 42, in accordance with an example of the present disclosure. Assembly 28 is shown still partially in sheath 54, so assembly 28 is not fully expanded yet. During this deployment state, shear forces due to assembly 28 being pressed against sheath 54 while being deployed may have impacted assembly 28, that sleeves 42 are to protect from.

As seen, assembly 28 comprises coated (46) electrodes 26 that are arranged as electrode pairs 262 disposed on splines 22. Electrode pairs 262 can be used to apply PFA between pairs of electrodes 26. Alternatively, any individual electrode 26 and or more than one electrode can be used for monopolar applying PFA between one or more electrodes and a return electrode on the patient's skin.

Sleeves 42 protect the electrodes from loosening due to shear forces during cathode deployment from and retraction into sheath 54. The sleeves have windows 62 that expose only the outer conductive surfaces. The thickness of sleeve 42 provides a raised edge around the window to protect coating 46 from scratching by spacing the surface away from a contact surface, e.g., sheath or heart cavity wall. Windows 62 can be prefabricated, or cut after positioning sleeves 42 on the electrodes. Shrink sleeves 42 can cover substantially the entire spline 22 or only a section in electrodes 26 vicinity.

In some examples, epoxy glue 64 is injected into the sleeve to bond electrodes 26 to spline 22. In other examples, the electrodes are first bonded on the spline, and then the shrink sleeve is added.

As noted above, in some applications, the sleeve can be per electrode, or it can be for more than one electrode.

Fig. 2 is a schematic pictorial illustration of the expandable basket assembly comprising electrode embedding sleeves 242, in accordance with another example of the present disclosure. Sleeves 242 have windows 162 that expose at least a portion of the outer conductive surfaces of individual electrodes 26.

The individual electrode windowing layout 126 may provide more resilience to the shear forces by wrapping each electrode 26 individually from both distal and proximal sides. Individual electrode windows 162 of windowing layout 126 can be prefabricated or cut after positioning sleeves 242 on the electrodes.

As noted above, in some applications, it might be useful to have the electrode's back surface exposed.

Fig. 3 is a schematic pictorial illustration of the expandable basket assembly comprising electrode embedding sleeves 342 that expose both the front and back sides of the electrodes, in accordance with an example of the present disclosure. As seen, each sleeve 342 has windows 362 and 366 that expose both the outer and back surfaces of electrodes pairs 262, respectively.

Figures 1-3 are brought by way of example where alternative designs are possible that serve similar functionalities. For example, the shape of the windows may differ from the shown rectangular shape in Fig. 2. As another example, the shape of sleeves of Fig. 3 may differ from that shown by adding sleeve portion between the electrodes that still maintains the windows in both the outer and back sides.

### METHOD OF FORMING SLEEVE-EMBEDDED ELECTRODES OF THE BASKET CATHETER

Fig. 4 is a flow chart that schematically illustrates a method of forming sleeve-embedded electrodes of the basket catheter of Fig. 1, in accordance with an example of the present disclosure. Preparation steps include (i) providing a shaft configured for insertion into an organ of a patient, and (ii) mounting a basket assembly at a distal end of the shaft, the basket assembly comprising a plurality of splines.

The method begins by mounting electrodes 26 on splines 22 of catheter distal end assembly 28, at electrode mounting step 402.

Next, a user or an automated tool bonds or otherwise fixates electrodes 26 to splines 22 of the catheter distal end assembly 28 at electrode fixating step 404.

Next, sleeves 42 are threaded over splines 22 such that they overlay electrodes 26 and portions of the splines at sleeves threading step 406.

The sleeves are then heated to shrink around the electrodes, as seen in Fig. 2, at sleeves fixing step 408.

At electrode-exposing step 410, windows are opened in the sleeves to expose the outer surface of the electrode (e.g., of electrode pairs). Alternatively, the windows may be formed before putting the sleeves over the electrodes.

The flow chart shown in Fig. 4 is chosen purely for the sake of conceptual clarity. The present example may comprise additional steps, such as applying epoxy to further bond the electrodes to the spline. This and other possible steps are omitted from the disclosure herein purposely to provide a more simplified flow chart.

### EXAMPLES

### Example 1

A medical probe includes a shaft, a basket assembly (28), and heat shrink sleeves (42, 242, 342). The shaft is configured for insertion into an organ of a patient. The basket assembly (28) is mounted at a distal end of the shaft, the basket assembly comprising a plurality of splines (22). A plurality of electrodes (26) is fitted on the plurality of splines (22), each electrode (26) defining an outer conductive surface (46). The heat shrink sleeves (42, 242, 342) are fitted over the electrodes, the sleeves comprising open windows (62, 162, 362, 366) that expose conductive surfaces (46) of the electrodes (26).

### Example 2

The medical probe according to example 1, wherein the plurality of electrodes (26) comprises one or more electrode pairs (262), and wherein a given sleeve (42, 342) is fitted over an electrode pair (262).

### Example 3

The medical probe according to any of examples 1 and 2, wherein the open windows (62, 162, 362) expose only outer conductive surfaces of the electrodes (26).

### Example 4

The medical probe according to any of examples 1 and 2, wherein the open windows (362, 366) expose both outer and back conductive surfaces of the electrodes (26).

### Example 5

The medical probe according to any of examples 1 through 4, and comprising epoxy glue (54) injected into a given sleeve (42, 242, 342) to bond the electrodes (26) onto the splines (22).

### Example 6

A medical probe manufacturing method, the method comprising providing a shaft A basket assembly (28) is mounted at a distal end of the shaft, the basket assembly comprising a plurality of splines (22). A plurality of electrodes (26) is fitted on the plurality of splines, each electrode defining an outer conductive surface (46). Heat shrink sleeves (42, 242, 342) are fitted over the electrodes (26), the sleeves comprising open windows (62, 162, 362, 366) that expose conductive surfaces (46) of the electrodes. The sleeves are heated to shrink them over the electrodes.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A medical probe, comprising:
a shaft for insertion into an organ of a patient;
a basket assembly (28) at a distal end of the shaft, the basket assembly (28) comprising a plurality of splines (22);
a plurality of electrodes (26) fitted on the plurality of splines, each electrode defining an outer conductive surface (46); and
heat shrink sleeves (42, 242, 342) fitted over the electrodes (26), the sleeves comprising open windows (62, 162, 362, 366) that expose conductive surfaces (46) of the electrodes (26).

2. The medical probe according to claim 1, wherein the plurality of electrodes (26) comprises one or more electrode pairs (262), and wherein a given sleeve (42, 342) is fitted over an electrode pair (262).

3. The medical probe according to any of claims 1 and 2, wherein the open windows (62, 162, 362, 366) expose only outer conductive surfaces (46) of the electrodes (26).

4. The medical probe according to any of claims 1 and 2, wherein the open windows (362, 366) expose both outer and back conductive surfaces (46) of the electrodes (26).

5. The medical probe according to any of claims 1 through 4, and comprising epoxy glue (54) injected into a given sleeve (42, 242, 342) to bond the electrodes (26) onto the splines (22).

6. A medical probe manufacturing method, the method comprising:
providing a shaft configured for insertion into an organ of a patient;
mounting a basket assembly (28) at a distal end of the shaft, the basket assembly comprising a plurality of splines (22) ;
fitting a plurality of electrodes (26) on the plurality of splines (22), each electrode defining an outer conductive surface (46);
fitting heat shrink sleeves (42, 242, 342) over the electrodes, the sleeves comprising open windows (62, 162, 362, 366) that expose conductive surfaces of the electrodes; and
heating the sleeves (42, 242, 342) to shrink them over the electrodes.

7. The method according to claim 6, wherein the plurality of electrodes (26) comprises one or more electrode pairs (262), and wherein fitting a given sleeve (42, 342) comprises fitting the sleeve over an electrode pair (262).

8. The method according to any of claims 6 and 7, wherein the open windows (62, 162, 362, 366) expose only outer conductive surfaces (46) of the electrodes (26).

9. The method according to any of claims 6 and 7, wherein the open windows (362, 366) expose both outer and back conductive surfaces (46) of the electrodes (26).

10. The method according to any of claims 6 through 9, and comprising injecting epoxy glue (54) into a given sleeve (42, 242, 342) to bond the electrodes (26) onto the splines (22).
